# EUROPEAN PATENT SPECIFICATION

(11) **EP 3 082 403 B1**
(45) Date of publication and mention of the grant of the patent: **15.05.2019**
(21) Application number: 13811572.0
(22) Date of filing: 20.12.2013
(51) Int. Cl.: A01H 5/00, C07K 14/415, C12N 15/82

(54) **ISOLATED NUCLEOTIDE SEQUENCE FROM SOLANUM LYCOPERSICUM FOR IMPROVED RESISTANCE TO TOMATO SPOTTED WILT VIRUS, TSWV.**
ISOLIERTE NUKLEOTIDSEQUENZ AUS SOLANUM LYCOPERSICUM ZUR ERHÖHTEN RESISTENZ GEGEN TOMATENBRONZEFLECKENVIRUS, TSWV.
SÉQUENCE DE NUCLÉOTIDES ISOLÉE PROVENANT DE SOLANUM LYCOPERSICUM POUR UNE RÉSISTANCE AMÉLIORÉE AU VIRUS DE LA TACHE BRONZÉE DE LA TOMATE (TSWV)

(43) Date of publication of application: 26.10.2016
(73) Proprietor: Isi Sementi S.p.A., 43036 Fidenza (IT)
(72) Inventor: BELFANTI, Enrico, I-61122 Pesaro (IT); MALATRASI, Marina, I-43055 Mezzani (IT); ORSI, Ilaria, I-42049 S. Ilario d'Enza (IT); BONI, Anna Giulia, I-43036 Fidenza (IT)
(74) Representative: Modiano, Micaela Nadia
(86) International application number: PCT/EP2013/077799
(87) International publication number: WO 2015/090468

(56) References cited:
- WO-A1-2013/127988
- US-A1- 2002 062 504
- US-A1- 2007 204 366
- C. LOPEZ ET AL: "Evolutionary analysis of tomato Sw-5 resistance-breaking isolates of Tomato spotted wilt virus", JOURNAL OF GENERAL VIROLOGY, vol. 92, no. 1, 1 January 2011 (2011-01-01), pages 210-215, XP055135247, ISSN: 0022-1317, DOI: 10.1099/vir.0.026708-0

### SEQUENCE LISTING

<110> ISI SEMENTI RESEARCH SOCIETA' A RESPONSABILITA' LIMITATA
<120> ISOLATED NUCLEOTIDE SEQUENCE FROM SOLANUM LYCOPERSICUM
<130> E052146 - M227216
<160> 5
<170> PatentIn version 3.5
<210> 1
   <211> 1163
   <212> DNA
   <213> Solanum lycopersicum
<400> 1
   actgcatagt tgccagactg tattcttcga actcactgtc aagatagtca aataaagcat 60
   ctttcacctc attccaccaa gattcttcca ttttcctttt tttgattatt ccagctacca 120
   agacaaccac taggggcagt cctttgcatt tttctgctac tgcttgactc acatcttgta 180
   gttcaggcgg gcaatcttcc ttttgaaaca cttttttctg caacaattgg caactctctt 240
   ctgttgtgag gaatggaaga gaataaggat cagtatggta cttgacttgc ttacccactt 300
   cttcaagtcg agttgttacg actattctgc ttctatttcc ggaatctgga aaggaaagcc 360
   ttaagtcatc ccataccata caatcccaca tatcatccaa tacaatgaga tatctctttc 420
   ccattaattt tctcctcaac atgtcggcaa caacgtcaac cgtagctcca ttgtcgttgc 480
   aacctgtaac ttgactgaaa atatcttgta ataactctct ccgattatac gtttgagaaa 540
   tgatgcacca tgctcgaaca tcaaagcgag aaacaataat gtcattattg tacaactttc 600
   tagcaattgt cgttttccct tgtcctccca tgcctacaat tgggacaacg tctagctcat 660
   ttgtacctct aatcagataa tcaattagtt cttctgcttt attctcaaaa cccactacct 720
   cctcatcagt tacaagattg ctatgtcgat ccggcagatg ttttaatgga gcagccacat 780
   agtgaggatt aagaggaatg tccgctgacc acatctcagt cacctctgca ttaatttgct 840
   tgatctcttt tacaattgta ggaagtgagc aaaaaatatg caaaaaagca ttatactgag 900
   caagaataga atcaatagca acctcagcct catatgccaa attgatggta cgcctctgaa 960
   gatctttagg aatgttatgt tcatggtgca cgacatcacg gaaaatggat gagagctcct 1020
   tctctaaaat ggatgtaaga gatgatagct ctttctccaa attccctaaa agaggtttca 1080
   tcaggaaacc taatccggac ttagatgtcg acatctcatt cagtttcctt aaaagagaat 1140
   ccagaaagct caatccacca aag 1163
<210> 2
   <211> 1163
   <212> DNA
   <213> Solanum lycopersicum cv Stevens
<400> 2
   actgcatagt tgccagactg tattcttcga actcactgtc aagatagtca aataaagcat      60
   ctttcacctc attccaccaa gattcttcca ttttcctttt tttgattatt ccagctacca     120
   agacaaccac taggggcagt cctttgcatt tttctgctac tgcttgactc acatcttgta     180
   gttcaggcgg gcaatcttcc ttttgaaaca cttttttctg caacaattgg caactctctt     240
   ctgttgtgag gaatggaaga gaataaggat cagtatggta cttgacttgc ttacccactt     300
   cttcaagtcg agttgttacg actattctgc ttctaattcc atcatctgga aaagaaagcc     360
   ttaagtcatc ccataccata caatcccaca tatcatccaa tacaatgaga tatctctttc     420
   ccattaattt tctcctcaac atgtcggcaa gaacatcaac cgtagctcca ttgtcgtcgg     480
   aacctgtaac ttgactgaaa atatcttgta ataactctct ccgattatac gtttgagaaa     540
   tgatgcacca tgctcgaaca tcaaagcgag aaacaataat gtcattattg tacaactttc     600
   tagcaattgt cgttttccct tgtcctccca tgcctacaat tgggacaacg tctagctcat     660
   ttgtacctct aatcagataa tcaattagtt cttctgcttt attctcaaaa cccactacct     720
   cctcatcagt tacaagattg ctatgtcgat ccggcagatg ttttaatgga gcagccacat     780
   agtgaggatt aagaggaatg tccgctgacc acatctcagt cacctctgca ttaatttgct     840
   tgatctcttt tacaattgta ggaagtgagc aaaaaatatg caaaaaagca ttatactgag     900
   caagaataga atcaatagca acctcagcct catatgacaa attgatggta cgcctctgaa     960
   gatctttagg aatgttatgt tcatggtgca cgacatcacg gaaaatggat gagagctcct    1020
   tctctaaaat ggatgtaaga gatgatagct ctttctccaa attccctaaa agaggtttca    1080
   tcaggaaacc taatccggac ttagatgtcg acatctcatt cagtttcctt aaaagagaat    1140
   tcagaaagct caatccacca aag                                           1163
<210> 3
   <211> 1163
   <212> DNA
   <213> Solanum lycopersicum cv Heinz 1706
<400> 3
   actgcatagt tgccagacta tattcttcga actcactgtc aagatagtca aataaagcat      60
   ctttcacctc attccaccaa gattcttcca ttttcctttt tttgattatt ccagctacca     120
   agacaaccac taggggcagt cctttgcatt tttctgctac tgcttgactc acatcttgta     180
   gttcaggcgg gcaatcttcc ttttgaaaca cttttttctg caacaattgg caactctctt     240
   ctgttgtgag gaatggaaga gaataaggat cagtatggta cttgacttgc ttacccactt     300
   cttcaagtcg agttgttacg actattctgc ttctaattcc aacatctgga aaagaaagcc     360
   ttaagtcatc ccataccata caatcccaca tatcatccaa tacaatgaga tatctctttc     420
   ccattaattt tctcctcaac atgtcggcaa gaacgtcaac cgtagctcca ttgtcgttga      480
   aacctgtaac ttgactaaaa atatcttgta ataactctct ctgattatac gtttgagaaa      540
   tgatgcacca tgctcgaaca tcaaagcgag aaacaataat gtcattattg tacaactttc      600
   tagcaattgt cgttttccct tgtcctccca tgcctacaat tgggacaacg tctagctcat      660
   ttgtacctct aatcagataa tcaattagtt cttctgcttt attctcaaaa cccactacct      720
   cctcatcagt tacaagattg ctatgtcgat ccggcagatg ttttaatgga gcagccacat      780
   aatgaggatt aagaggaatg tccgctgacc acatctcagt cacctctgca ttaatttgct      840
   tgatctcttt tacaattgta ggaagtgagc aaaaaatatg caaaaaaaca ttatactgag      900
   caagaataga atcaatagca acctcagcct cacatgccaa attgatggta cgtctctgaa      960
   gatctttagg aatgttatgt tcatggtgca cgacatcact gaaaatggat gagagctcct     1020
   tctctaaaat ggatgcaaga gatgatagct ctttctccaa attccctaaa agaggtttcg     1080
   tcaggaaacc taatccggac ttagatgtcg acatctcatt cagtttcctt aaaagagaat     1140
   ccagaaagct caatccacca aag                                             1163
<210> 4
   <211> 19
   <212> DNA
   <213> Artificial Sequence
<220>
   <223> Forward primer
<400> 4
   actgcatagt tgccagact 19
<210> 5
   <211> 22
   <212> DNA
   <213> Artificial Sequence
<220>
   <223> Reverse primer
<400> 5
   ctttggtgga ttgagctttc tg 22

## Description

The present invention relates to a nucleotide sequence that is responsible for the resistance of tomato plants to variants of the Tomato Spotted Wilt Virus (TSWV). More specifically, the present invention discloses a new sequence in the SW-5 locus.

TSWV (Tomato Spotted Wilt Virus) is a plant infecting virus belonging to the *Tospovirus* genus of the *Bunyaviridae* family. Its genome consists of three negative-sense or ambisense RNA segments: segment L (large) encoding a putative RNA dependent RNA polymerase (de Haan et al., 1991), segment M (medium) encoding the cell-to-cell movement protein NSm (Li et al., 2009) and the precursor of the surface glycoproteins GN/GC, involved in TSWV transmission by thrips (Sin et al., 2005; Naidu et al., 2008) and segment S (small) encoding a silencing suppressor, NSs (Takeda et al., 2002) and the nucleocapsid, N (de Haan et al., 1990).

Tospoviruses are the sole plant infecting viruses in this family, as all other described members of the *Bunyaviridae* family infect animals. The Tospovirus genus takes its name right from the *Tomato Spotted Wilt Virus* (TSWV), discovered in Australia in 1915. TSWV remained the only member of the family until the early 1990s when genetic characterization of viruses discovered in plants became more common. There are now at least twenty viral species in the family with more being recorded and described on a relatively regular basis. Together, these viruses have been documented infecting over eight hundreds different plant species from 82 different families.

TSWV has a host range spanning several hundred species in both monocotyledonous and dicotyledonous plants, among which are vegetables and ornamental and weedy plants; in particular, TSWV infections represent a severe problem affecting worldwide the production of, *inter alia,* pepper, tomato, artichoke, lettuce, aubergine, peanut, tobacco. In particular, in some areas of the world TSWV has become a limiting factor in tomato production (Canady et al., 2001), causing a loss in tomato production of up to 70%.

At least ten species of tiny insects known as thrips, belonging to the *Thripidae* family are known as vectors for the transmission of at least thirteen tospoviruses, among which is TSWV. TSWV is spread in particular by the tobacco thrips (*Frankliniella fusca*) and the western flower thrips (*Frankliniella occidentalis*)*.* Tobacco thrips vectors dispersing from weed hosts are able to transmit TSWV to susceptible crops quickly, often before they can be killed by insecticides applied to the crop. Western flower thrips are extremely difficult to eradicate, as they are highly tolerant to the insecticides commonly used on tomato plants and as they prefer to reside deep within the blossoms where they can hardly be reached by insecticides. Additionally, western flower thrips infestations are exacerbated by application of certain insecticides that eliminate natural enemies of thrips.

On tomato, the first symptoms of TSWV infection usually are small, orange-coloured flecks on some middle or lower leaves or on the calyx. As new spots appear, older leaves turn brown, die, and droop. Similar spots or streaks occur on the stems and petioles. The entire plant becomes dwarfed, and with its drooping leaves it resembles a plant affected by a wilt. On the green fruits, yellowish spots up to 10 mm in diameter appear, usually with distinct concentric zones of shades of yellow or brown alternating with green and later with pink or red.

Controlling TSWV infection by agronomic or chemical means is not particularly effective, because of several matters such as: the difficulty of an early diagnosis of the infection, the lack of the possibility to cure infected plants, the wide number of vector insects, the diffusion of insecticide-resistant thrips, the wide host tropism of TSWV (over 1000 plant species).

Most TSWV strains are not able to damage tomato varieties containing the Sw-5b allele of the SW-5b gene. SW-5b is a single dominant resistance gene, originally originating from the tomato specie *Lycopersicon peruvianum* and locating on chromosome 9. Only two allelic variants of the SW-5b tomato gene are known nowadays, namely sw-5b, i.e. the sensitive variant to TSWV and Sw-5b, i.e. the resistant variant to TSWV. In the context of the present invention, "sw-5b" indicates the TSWV-sensitive allelic variant of the SW-5b gene and "Sw-5b" indicates the TSWV-resistant allelic variant of the SW-5b gene.

It has been suggested (Brommonschenkel et al., 2000) that the Sw-5b protein is a cytoplasmic protein, with a potential nucleotide binding site domain structured as a coiled coil and a carboxy-terminal end consisting of leucine-rich repeats.

Tomato plants having at least one Sw-5b allele therefore represent an utmost benefit for growing in areas wherein the presence of TSWV is endemic. For this reason, among the tomato genes which confer a resistance to TSWV, nowadays only SW-5b has a relevance in commercial tomato cultivars. The use of TSWV-resistant tomato cultivars represents the most important strategy for the control of TSWV. In addition, the relevance of the TSWV-resistant allele Sw-5b lays in its durability to multiple tospoviruses, further to TSWV (Boiteux and Giordano, 1992; Stevens et al, 1992).

However, in recent times, in areas where Sw-5b tomato cultivars were grown, new TSWV strains have been identified that overcome the resistance granted by Sw-5b (Latham. and Jones, 1998; McMichael et al. 2002). These TSWV strains are commonly referred to as "resistance-breaking" strains (RB). In particular, in 2005 Ciuffo et al. recorded the presence in Italy of a Sw-5b resistance-breaking strain, which was named T1012.

For this reason, resistant tomato cultivars usually have to be re-evaluated annually for their resistance to TSWV.

Therefore, finding new means by which resistance to "resistance-breaking" TSWV strains can be conferred to tomato plants represents an extremely relevant goal. In particular, there is the need for finding further genes and/or further alleles of known genes allowing tomato plants not to be affected both by Sw-5b-sensitive TSWV and by "resistance-breaking" TSWV.

US2002/062504 relates to an isolated plant nucleic acid which confers resistance to Tospoviruses as well as expression systems, host cells, and transgenic plants transformed with such a nucleic acid.

WO2013/127988 relates to a pepper plant which is resistant against the Tsw-resistance breaking strain of TSWV designated Ve427^{RB}.

The main purpose of the present invention is to provide a new tomato nucleotide sequence which makes plants resistant to infections mediated by tospoviruses, in particular by TSWV. Within this purpose, a scope of the invention is to provide a tomato nucleotide sequence which grants plants an at least moderate (i.e. intermediate) resistance to TSWV "resistance-breaking" strains (i.e. mutant strains which are capable to infect plants having the Sw-5b allele), while maintaining a standard (i.e. high) resistance to TSWV strains which are not capable to infect plants having the Sw-5b allele.

As it is well known in the field of plant genetics, resistance is the ability of a plant variety to restrict the growth and development of a specified pathogen able to attack said plant and/or to reduce the damage such pathogen may cause, when compared to susceptible plant varieties under similar environmental conditions and pathogen pressure. Resistant varieties may however exhibit some disease symptoms and/or damage under heavy pathogen pressure. Two levels of resistance are defined:
(i) high resistance (HR): high resistance plant varieties are plant varieties which highly restrict the growth and development of a specified pathogen under normal pathogen pressure when compared to susceptible varieties. These plant varieties may, however, exhibit some symptoms and/or damage under heavy pathogen pressure;
(ii) intermediate resistance (IR): intermediate resistance plant varieties are plant varieties which restrict the growth and development of a specified pathogen but may exhibit a greater range of symptoms and/or damage compared to high resistance varieties. Intermediately resistant plant varieties will still show less severe symptoms and/or damage than susceptible plant varieties when grown under similar environmental conditions and/or pathogen pressure. The above definitions in regard to resistance are in accord to ISF (International Seed Federation).

The above purpose and scopes, as well as further scopes which will be better clarified in the following, are reached by an isolated nucleotide sequence comprising SEQ. ID. No: 1, or comprising complementary sequences thereof.

The above purpose and scopes are also reached by the use of a nucleotide sequence comprising SEQ. ID. No: 1, or comprising complementary sequences thereof for marker assisted selection of plants.

Further scopes, features and advantages of the present invention will better appear from the detailed description provided in the following.

The expression "parental line" is commonly used to refer to a phenotypically uniform plant population having a high degree of homozygosity. A typical strategy for developing new parental lines of tomato plants (a mainly autogamous species) is represented by the selection among the plant population which has a genetic variability for one or more phenotypic character(s). This population can be generated by cross-breeding two parental lines; the offspring obtained in this way is named "a hybrid": a hybrid is uniform from a phenotypical point of view but it is scarcely homozygous from a genotypical point of view. By self-fecundation of an hybrid, a so-called F2 population is obtained, wherein the characters which originally were typical of one or the other of the parental lines have recombined. The F2 population is thus non-uniform from a phenotypical point of view and has an intermediate degree of homozygosity from a genotypical point of view.

As stated above, nowadays only two allelic variants of the SW-5b gene are known, i.e. sw-5b and Sw-5b. Surprisingly, it has been found that a third allele of the SW-5 tomato gene exists, which has been named Sw-5b2 and which is distinct from both the Sw-5b and the sw-5b allelic variants known up to now. The Sw-5b2 allele has been retrieved in the Applicant's germplasm collection. The Sw-5b2 allele is a nucleotide sequence comprising SEQ. ID. No: 1, or comprising a complementary sequence thereof.

Therefore, in the context of the present invention, the term "Sw-5b2 allele" is meant to indicate a nucleotide sequence which comprises SEQ. ID. No: 1 or comprises a sequence complementary to said SEQ. ID. No: 1. Sw-5b2 is allelic to both Sw-5b and sw-5b (i.e. Sw-5b2, Sw-5b and sw-5b occupy exactly the same position in the tomato genome). Such SEQ. ID. No: 1 has been isolated and amplified through a couple of primers designed ad hoc by the inventor. These primers are specific for the SW-5b gene, that is, they are capable of assessing the presence of the Sw-5b, sw-5b and Sw-5b2 alleles. These primers have the following nucleotide sequences:
Forward primer (Sw5 30937 F):
   ACTGCATAGTTGCCAGACT (SEQ. ID. No: 4)
Reverse primer (Sw5 32077 R):
   CTTTGGTGGATTGAGCTTTCTG (SEQ. ID. No: 5)

Once isolated, the nucleotide sequence corresponding to SEQ. ID. No: 1 has been sequenced. The sequencing showed that SEQ. ID. No: 1 is identical to the corresponding nucleotide sequence in the Sw-5b allele (i.e. SEQ. ID. No: 2) but 11 nucleotides. Similarly, SEQ. ID. No: 1 is identical to the corresponding nucleotide sequence in the sw-5b allele (i.e. SEQ. ID. No: 3) but 17 nucleotides.

The demonstration that Sw-5b2 is allelic with respect to both Sw-5b and sw-5b is provided by the fact that on about 400 tomato plants of the F2 population studied, no plants have been found which had, at the same time, the Sw-5b2 sequence, the Sw-5b sequence and the sw-5b sequence. Another confirmation is given by the fact that on about 400 tomato plants of the F2 population studied, no recombinants were found. Moreover, analyzing the PCR products by the "high resolution melting curve" technique, the presence of three alleles in a single plant was never found. The above evidence therefore demonstrate that the Sw-5b2, Sw-5b and sw-5b sequences are allelic.

The resistance to infection by "resistance-breaking" TSWV strains in tomato plants having the Sw-5b2 allele may be due to the fact that the nucleotide differences result in a polypeptide encoded by Sw-5b2 differing from the polypeptide encoded by Sw-5b in its secondary and tertiary structure and, consequently, in its function.

Tomato plants have been artificially infected with two TSWV strains: T105 TSWV, which is the wild-type strain of TSWV, widely spread in Italy and T1012 TSWV, which is a "resistance-breaking" strain isolated in Italy. Three kinds of plants have been infected: (i) plants containing only the sw-5b (sensitive) allele; (ii) plants containing the Sw-5b (resistant) allele and (iii) plants containing the Sw-5b2 allele. These studies have been carried out at the Vegetal Virology Institute of the National Centre for Research (CNR) in Turin. Three distinct infection cycles have been performed.

Tomato plants containing only the sw-5b (sensitive) allele undergo a letal infection by both T105 and T1012 TSWV strains.

Tomato plants containing the Sw-5b (resistant) allele survive to infection by T105 TSWV (wild-type) strain but die when infected with T1012 TSWV (resistant) strain.

Finally, all tomato plants containing the Sw-5b2 allele survive to infection by T105 TSWV (wild-type) strain and a great percentage thereof survive also to infection by T1012 TSWV (resistant) strain. The survival degree varies from 100% to 20% (the average being about 60%) according to the tomato plant variety, the zygosis condition, the inoculus pressure.

The Sw-5b2 allele has been detected by the PCR technique using the forward primer Sw5 30937 F and the reverse primer Sw5 32077 R described hereinabove and the SsoFast EvaGreen Supermix (Bio Rad, catalog #172-5205). The resulting product has been analyzed by "high resolution melting curve" in a Bio Rad cfx-96 Real-time system. The PCR product was purified by "PureLink Quick Gel Extraction Kit" of Invitrogen (catalog #2100-12) after a run into agarose gel. The cloning has been made by Topo PCR Cloning (Invitrogen catalog #450030) and the sequencing has been made by Synergene Biotech GmbH (Switzerland) .

The above results demonstrate that the Sw-5b2 allele is of great relevance for granting tomato plants an almost moderate resistance to infections by TSWV strains which are harmful for plants having the Sw-5b allele (i.e. to "resistance-breaking" TSWV strains).

Another aspect of the present invention is represented by the use of a nucleotide sequence comprising SEQ. ID. No: 1, or comprising a complementary sequence thereof, for marker assisted selection of plants.

Marked assisted selection (MAS, also known as marker aided selection) is an indirect selection process, widely used in plant and animal breeding, whereby a genetic character of interest (e.g., productivity, disease resistance, stress tolerance, sterility, etc.) is selected, not on the basis of the character itself, but on the basis of a marker linked to it. The principle at the basis of MAS is that the marker used for selection associates at high frequency with the gene (or the genetic locus) of interest, due to genetic linkage, i.e. the close proximity, on the chromosome, of the marker locus and the character of interest-determining locus. Accordingly, the gene of interest and the marker tend to move together during segregation of gametes due to their proximity on the same chromosome and low probability of chromosome crossover events between the marker and gene of interest.

Generally speaking, markers can be of one of three types: (i) morphological (such as, as far as plants are concerned, leaf coloration, height, grain color), (ii) biochemical (i.e. a protein that can be extracted and observed, such as an enzyme), or (iii) DNA-based and/or molecular, whereby an easily identifiable gene (or DNA sequence) occurs in proximity to the gene or locus of interest. The best possible marker is located on the gene that determines the character of interest.

MAS can be very useful to efficiently select for characters that have a recessive phenotype, are difficult or expensive to measure, are expressed late in development and/or require for the presence of special conditions in order to be expressed, like the breeding for disease and pest resistance.

MAS allows to confirm, at a certain point in the breeding process, that the selected individuals or they progeny express the desired phenotype or character, thus developing new parental lines and, successively, new hybrids, where the character of interest is present.

In this invention, SEQ. ID. No: 1 represents the marker which allows to identify the presence of the Sw-5b2 allelic variant, corresponding to the phenotype of plants resistant to infection by "resistance-breaking" TSWV strains. The use of SEQ. ID. No: 1 as a marker to identify the presence of the Sw-5b2 allelic variant offers a series of advantages over infecting (either artificially or naturally) the plants. First, the resistance to infection by "resistance-breaking" TSWV strains can be assessed in a more reliable way and within a shorter time (few days), as it is not necessary to develop adult plants. In addition, the use of SEQ. ID. No: 1 as a marker for selection is nondestructive for the plants and it even prevents the spoiling of the tested plants. Furthermore, since no plant infection is performed, such use allows to avoid the risk of accidental contamination of non-infected plants. This use has also the advantage of allowing to distinguish whether there is homozygosity or heterozygosity for the Sw-5b2 allelic variant in the tested plants. This is a very important feature in that the progeny of plants heterozygous for the Sw-5b2 allele would not be stable in terms of resistance to infection by "resistance-breaking" TSWV strains. Accordingly, also economic advantages follow from the above: the possibility to precociously identify plants homozygous for the Sw-5b2 allele (which amount to 25%) allows to discard the part of the plant progeny (i.e. 75%) which is not homozygous for the Sw-5b2 allele, thus saving time, money and work load.

In a preferred embodiment, the invention is directed to the use of a nucleotide sequence comprising SEQ. ID. No: 1 or comprising a complementary sequence thereof, for marker assisted selection of plants having a commercial relevance (either ornamental plants or vegetables). Examples of commercially relevant plants are the following: tomato, tobacco, sweet pepper, hot pepper, eggplant. A more preferred embodiment is the use of a nucleotide sequence comprising SEQ. ID. No: 1 or comprising a complementary sequence thereof, for marker assisted selection of tomato plants.

An aspect of the present disclosure is represented by the use of a nucleotide sequence comprising SEQ. ID. No: 1, or comprising a complementary sequence thereof, for introgression into plants.

Introgression, also known as introgressive hybridization, is a process by which a gene of interest is moved from the genetic pool of an individual which contains the gene, into the gene pool of another individual which does not contain it. Introgression may occur either by means of a traditional technique, i.e. through repeated back-crossings between the two individuals, or by means of a biotechnological technique, for example by genetic transformation.

In particular, the nucleotide sequence comprising SEQ. ID. No: 1, or comprising a complementary sequence thereof, can be introgressed into species that do not contain this sequence, but are commercially relevant by virtue of one or more commercially valuable characteristics (such as high productivity, pest resistance, notable flowering etc.). Accordingly, new parental lines having both the commercially valuable characteristics and the resistance to infection by "resistance-breaking" TWSV strains (conferred by the presence of the Sw-5b2 allele) can be obtained.

In a preferred aspect, the present disclosure regards the use of a nucleotide sequence comprising SEQ. ID. No: 1 or comprising a complementary
sequence thereof for introgression into plants having a commercial relevance (either ornamental plants or vegetables). Examples of commercially relevant plants are the following: tomato, tobacco, sweet pepper, hot pepper, eggplant. A more preferred aspect of the present disclosure is the use of a nucleotide sequence comprising SEQ. ID. No: 1 or comprising a complementary sequence thereof, for introgression into a tomato plant.

Furthermore, another aspect of the present disclosure is represented by a method to make plants almost moderately resistant to infections by TSWV strains which are harmful for plants having the Sw-5b allele by introducing into the plant genome a nucleotide sequence comprising SEQ. ID. No: 1 or comprising a complementary sequence thereof.

In a preferred aspect of the disclosure, the plants which can be made resistant to infections by "resistance-breaking" TSWV strains are plants having a commercial relevance (either ornamental plants or vegetables). More preferably, the commercially relevant plants are the following: tomato, tobacco, sweet pepper, hot pepper, eggplant. A still more preferred aspect of the present disclosure is a method to make tomato plants resistant to infections by "resistance-breaking" TSWV strains.

In view of the above, it has been established that the present invention fully accomplishes the purpose and the scopes put forward hereinabove.

In fact, nucleotide sequences comprising SEQ. ID. No: 1 or comprising a complementary sequence thereof proved to provide an increase in the resistance of tomato plants to infection by TSWV. In particular, tomato plants having in their genome a sequence comprising SEQ. ID. No: 1 or comprising a complementary sequence thereof, present the same degree of resistance to wild-type TSWV as tomato plants having SEQ. ID. No: 2 (i.e. having the Sw-5b allele). The resistance degree assured by the Sw-5b allele (and, accordingly, also by the Sw-5b2 allele) towards wild-type TSWV is known to be a standard degree (i.e. an elevate resistance).

Further to offering the above standard resistance to wild-type TSWV, a nucleotide sequence comprising SEQ. ID. No: 1 or comprising complementary sequences thereof also endorses tomato plants with a moderate resistance to mutant TSWV strains ("resistance-breaking" TSWV strains), which plants having the Sw-5b allele are sensitive to.

SEQ. ID. No: 1 is therefore of the utmost importance from an economical point of view. In fact, the presence of a nucleotide sequence comprising SEQ. ID. No: 1 or comprising complementary sequences thereof in commercially relevant plants (such as vegetables or ornamental plants) actually shelters growing of such plants, allowing a more efficient growth and yielding greater quantities of products.

## Claims

1. An isolated nucleotide sequence comprising SEQ. ID. No: 1, or comprising complementary sequences thereof.

2. Use of the nucleotide sequence of claim 1 for marker assisted selection of tomato plants.

## Patentansprüche

1. Eine isolierte Nukleotidsequenz, die Sequenzidentifikationsnr. 1 oder komplementäre Sequenzen davon umfasst.

2. Nutzung der Nukleotidsequenz gemäß Anspruch 1 zur markergestützten Auswahl von Tomatenpflanzen.

## Revendications

1. Séquence de nucléotides isolée comportant la SEQ ID N° 1, ou comportant des séquences complémentaires de celle-ci.

2. Utilisation de la séquence de nucléotides de la revendication 1 pour la sélection assistée par marqueurs de plants de tomates.
